# EUROPEAN PATENT SPECIFICATION

(11) **EP 2 274 024 B1**
(45) Date of publication and mention of the grant of the patent: **15.05.2013**
(21) Application number: 09726890.8
(22) Date of filing: 26.03.2009
(51) Int. Cl.: A61L 27/50

(54) **BIOCOMPATIBLE PRODUCTS FOR MAGNETIC PARTICLE IMAGING**
BIOKOMPATIBLE PRODUKTE ZUR ABBILDUNG MAGNETISCHER PARTIKEL
PRODUITS BIOCOMPATIBLES POUR IMAGERIE DE PARTICULES MAGNÉTIQUE

(30) Priority: 03.04.2008 EP 08103358
(43) Date of publication of application: 19.01.2011
(73) Proprietor: Koninklijke Philips Electronics N.V., 5621 BA Eindhoven (NL)
(72) Inventor: MARKOV, Denis, 5656 AE Eindhoven (NL); BOEVE, Hans, M., B., 5656 AE Eindhoven (NL)
(74) Representative: Van Velzen, Maaike Mathilde
(86) International application number: PCT/IB2009/051274
(87) International publication number: WO 2009/122330

(56) References cited:
- EP-A1- 1 738 773
- US-A1- 2004 030 379
- US-B1- 6 808 535
- NASONGKLA NORASED ET AL: "Multifunctional polymeric micelles as cancer-targeted, MRI-ultrasensitive drug delivery systems" NANO LETTERS, ACS, WASHINGTON, DC, US LNKD- DOI:10.1021/NL061412U, vol. 6, no. 11, 1 November 2006 (2006-11-01), pages 2427-2430, XP002540355 ISSN: 1530-6984 [retrieved on 2006-10-24]

## Description

### FIELD OF THE INVENTION

The present invention relates to materials and methods for non-invasive imaging of biocompatible products *in vivo* and *in vitro.*

### BACKGROUND OF THE INVENTION

Biocompatible products are employed in a multitude of biomedical applications. For example, biocompatible products include artificial tissue constructs, microcarriers or microcontainers as well as implants. However, to date, imaging techniques are rarely used for visualizing or monitoring, for example, remodelling, development or biodegradation of such products *in vivo* and *in vitro.* Computed tomography, magnetic resonance imaging (MRI) or ultrasound imaging have been suggested and can be utilized for such monitoring each having their disadvantages. Generally, contrast agents are used to increase the sensitivity of these techniques.

A method for monitoring remodelling of an artificial construct using signal enhancing agents and magnetic resonance imaging (MRI) is described in US 2006/0204445. However, contrast enhanced MRI senses a proton relaxation of water (rarely also lipids) in the magnetic field effected by a contrast agent. Therefore, only a contrast agent at the very interface between the biocompatible product and a body fluid or tissue will contribute to signal enhancement. This results in dramatic limitation of the signal to noise ratio. Alternatively, a contrast agent can be encapsulated with water in the bulk of the biocompatible product, which would improve the signal to noise ratio but would complicate a tissue construction and limit the number of its applications. EP 1 738 773 A1 relates to complexes which contain polycrystalline magnetic iron oxide particles in a pharmaceutically acceptable shell and to its use in magnetic particle imaging (MPI).

### SUMMARY OF THE INVENTION

In many biomedical applications it is essential to visualize biocompatible materials *in vitro* and *in vivo.*

One main field in biomedical applications regards tissue engineering which involves development of therapeutic strategies aiming at the replacement, repair, maintenance or enhancement of tissue function. For example, by seeding artificial tissue constructs such as scaffolds with cells, tissue is grown *in vitro* or *in vivo* to restore damaged tissue. During the process of tissue formation, the tissue is developed and then remodelled to become more like native tissue. However, a tissue-remodelling that takes place too slowly can lead to a pathologic response of surrounding tissues and compliance mismatch of the vessel, while rapid remodelling can result in premature failure of the engineered construct. Moreover, biodegradability is often an essential factor since artificial tissue constructs should preferably be absorbed by the surrounding tissue avoiding the necessity of a surgical removal. The rate at which degradation occurs has to coincide as much as possible with the rate of tissue formation. Thus, it would be advantageous to provide a material or method that allows to monitor development, remodelling and biodegradation of biocompatible materials such as artificial tissue constructs *in vitro* and *in vivo.*

A major problem in tissue engineering is the availability of a sufficient number of cells with the appropriate phenotype for delivery to damaged tissue. Commonly, this difficulty is overcome by using bioreactor culture systems for cell amplification which employ microcarriers and/or microcontainers. Besides serving as substrates for the propagation of anchorage-dependent cells, microcarriers and/or microcontainers can also be used to deliver the expanded undifferentiated or differentiated cells to the site of the defect or to deliver active agents in a living organism. However, the efficiency of the targeted delivery of cells or active agents depends greatly on the imaging procedures used. Therefore, it would be advantageous to provide a method for monitoring the targeted delivery of cells or active agents *in vitro* and *in vivo.* Furthermore, it would be advantageous to provide microcontainers that enables improved targeted delivery of cells or active agents to a target site.

Another class of widely used biocompatible products are implants. Especially in minimally-invasive surgeries, correct placement and orientation of the particular implant is difficult. Commonly, visibility of minimally-invasive procedures is accomplished through the use of endoscopes, or other such insertable magnification and/or illumination devices, wherein the image generated by an endoscope can be viewed through some type of ocular, or on a video display screen adjacent the surgery. Alternatively, many surgeries, especially graft implant procedures, utilize radiopaque markers on the implant and an external imager. Although great advances have been made in this respect, these systems are relatively expensive and all require sterilization or shielding in the operating room. Therefore, there is still a need for an improved method that enables monitoring of biocompatible materials after they have been introduced into a living organism. Furthermore, it would be advantageous to provide a biocompatible product that can be located or detected after it has been introduced in the desired target site.

To better address one or more of the above-mentioned needs or objects, in a first aspect of the invention a use of a MPI tracer agent for visual monitoring a biocompatible product comprising at least one magnetic particle imaging (MPI) tracer agent using magnetic particle imaging is provided, wherein the biocompatible product is selected from the group consisting of a microcontainer comprising a rigid or semi-rigid material, an artificial tissue construct and an implant.

The term "biocompatible product" as used herein refers to an artificial product that does not cause toxic or injurious effects on biological systems.

The term "scaffold" as used herein, means a material having an extended repeating structure, which forms a framework or matrix onto which and into which additional components may be introduced to impart additional features to the material.

The term "biodegradable" as used herein refers to a material that can be absorbed or degraded by a living organism.

The term "microcontainer" as used herein refers to a rigid or semi-rigid, optionally porous material suitable to support cell culture, in which the cells can be shielded from the environment for at least some time during application such as cell handling or cell delivery.

The term "target site" is defined for the purposes of the present invention as the location, where the biocompatible product should have its effect, e.g. to replace damaged tissue. The target side can comprise a location *in vitro,* e.g., in a Petri dish, or *in vivo,* e.g. in a living organism.

The term "implant" as used herein refers to an object or material inserted or grafted into a living organism for prosthetic, therapeutic, diagnostic, or experimental purposes.

The term "MPI tracer agent" as used herein refers to a particulate material that can be employed in the magnetic particle imaging (MPI) method which is, for example, described in DE 10151778.

A "pharmaceutically acceptable shell" within the context of the present invention is a layer of a substance or a substance mixture which covers the core of the MPI tracer agent in such a way that, when administered to a patient, life-threatening side effects do not arise.

These and other aspects of the invention will be apparent from and elucidated with reference to the embodiments described hereinafter.

### DETAILED DESCRIPTION OF EMBODIMENTS

According to one embodiment of the present invention a biocompatible product is provided comprising at least one magnetic particle imaging (MPI) tracer agent. It has been found that enhanced or good signal-to-noise ratio can be obtained with magnetic particle imaging (MPI) techniques which are, for example, described in DE 10151778. In this method, a magnetic field having a spatial distribution of the magnetic field strength is generated such that a first sub-zone having a relatively low magnetic field strength and a second sub-zone having a relatively high magnetic field strength are formed in the examination area, wherein magnetic particles have been introduced into the examination area. The position in space of the sub-zones in the examination area is then shifted, so that the magnetization of the particles in the examination area changes locally. Signals are recorded which are dependent on the magnetization in the examination area, and by extracting information concerning the spatial distribution of the magnetic particles in the examination area an image thereof can be established. Thus, this method offers the possibility of non-invasive imaging with a high spatial resolution.

In one embodiment the biocompatible product is suitable for introduction into a living organism. The living organism can be a microorganism, a plant or a mammal, e.g., a human, monkey, dog, cat, mouse, rat, cow horse, pig, goat or sheep.

The biocompatible products of the present invention can be manufactured from any material that does not cause toxic or injurious effects on biological systems. Preferably, materials are used that are standard or commonly used materials for the respective biocompatible product. Examples include synthetic and natural polymers, precious metals, titanium and other metals, porcelain, alumina and other ceramics.

According to one embodiment, the biocompatible product is at least partially biodegradable.

Representative biodegradable organic materials may include natural or synthetic polymers, e.g., collagen, cellulose, silicone, poly(alpha esters) such as poly(lactate acid), poly(glycolic acid), polyorthoesters or polyanhydrides. In an exemplary embodiment the biodegradable material comprises a hydrogel. Hydrogels can be made biodegradable with a wide range of degradation times which may be useful, e.g., in tissue engineering, cell therapy applications or controlled release of pharmaceutically active agents.

Examples for biodegradable inorganic materials are metals or alloys based on at least one of magnesium or zinc, or alloys comprising at least one of Mg, Ca, Fe, Zn, Al, W, Ln, Si, or Y. Also suitable are, e.g., alkaline earth metal oxides or hydroxides, such as magnesium oxide, magnesium hydroxide, calcium oxide, and calcium hydroxide or mixtures thereof. In an exemplary embodiment of the invention, the biocompatible product can also comprise inorganic composites or organic composites or hybrid inorganic/organic composites.

In one embodiment, the biocompatible product is an artificial tissue construct. The artificial tissue construct can be, e.g., a scaffold providing a supportive framework that allows cells to attach to it, and grow to it. Typically, a scaffold can be placed in damaged tissue areas with the aim of inducing growth of cells from the surrounding healthy tissue to restore damaged tissue. In an exemplary embodiment the scaffold has a three-dimensional structure of interconnected pores to permit cell attachment.

Examples of synthetic polymers suitable for artificial tissue constructs comprise, e.g, poly(urethanes), poly(siloxanes) or silicones, poly(ethylene), poly(vinyl pyrrolidone), poly(2-hydroxy ethyl methacrylate), poly(N-vinyl pyrrolidone), poly(methyl methacrylate), poly(vinyl alcohol) (PVA), poly(acrylic acid), poly(vinyl acetate), polyacrylamide, poly(ethylene-co-vinyl acetate), polyethylene glycol), poly(methacrylic acid), polylactic acid (PLA), polyglycolic acids (PGA), poly(lactide-co-glycolides) (PLGA), nylons, polyamides, polyanhydrides, poly(ethylene-co-vinyl alcohol) (EVOH), polycaprolactone, poly(vinyl acetate), polyvinylhydroxide, poly(ethylene oxide) (PEO) and polyorthoesters or any combination thereof.

A commonly used synthetic biodegradable material for an artificial tissue construct is polylactic acid (PLA), a polyester which can degrade within the human body to form lactic acid, a naturally occurring chemical which can be easily removed from the body. Similar materials are polyglycolic acid (PGA) and polycaprolactone (PCL) which posses a degradation mechanism similar to that of PLA, but exhibit respectively a faster and a slower rate of degradation compared to PLA. The artificial tissue construct can also be made from natural materials, e.g. proteic materials, such as collagen or fibrin, and polysaccharidic materials, like chitosan or glycosaminoglycans (GAGs). Furthermore, the artificial tissue construct can comprise a decellularized matrix, i.e. a biostructure such as an organ or part thereof from which the cellular and tissue content has been removed by artificial methods leaving behind an intact acellular infrastructure. Decellularized matrices can be rigid, or semi-rigid, having an ability to alter their shape.

In another embodiment the biocompatible product is a microcontainer. The microcontainer can permit cell attachment and can shield the cells from the environment for at least some time during application, e.g. cell handling or cell delivery. The microcontainer can be a planar, two-dimensional object ("flake"), wherein this object comprises a rigid or semi-rigid, optionally porous material which, upon application of an extrinsic stimulus, e.g. heating, change of pH or exposure to electromagnetic waves, is transferred from the planar state into a rolled state. The shape of the flake can resemble, e.g., a square, a rectangle or a parallelogram. Likewise, the flake can also have a circular, elliptic, trapezium like, hexagonal, polygonal or triangular shape. Typical dimensions of the flake are in the order of the dimensions of the cells or somewhat bigger than that, e.g., the size or the length of such a flake is from 10 µm to 100 mm. The thickness of the flake can be, e.g., from 100 nm to 1 mm. The rolled state can comprise, e.g., a bilayer, trilayer, multilayer or gradient structure. For example, the rolled state comprise an at least partially multilayered cylinder or a cylindrical body just substantially closed. Preferably, the cells are only present on the flakes and not on the substrate in between the flakes. However, it can also be desired that the cells are present in the interstitium between the flakes. The release of the cells inside a living organism can happen in a controlled way by controlling the transfer between the rolled and the planar state of the flake.

The microcontainer can be manufactured from a material which is biodegradable and biocompatible. For example, a hydrogel material may be suitable. Typical hydrogel materials may include, e.g., poly(meth)acrylic materials, substituted vinyl materials or mixtures thereof, as well as epoxydes, oxetans or thioles. Other suitable materials include, e.g., poly(glycolic acid), poly(lactic acid) and their copolymers and derivates. Other materials that can be employed can comprise, e.g., alginate, hyaluronic acid, chitosan, collagen, gelatin, silk or combinations thereof.

In a further embodiment the biocompatible product is an implant.

Examples of implants may comprise, e.g., vascular endoprostheses, intraluminal endoprostheses, stents such as coronary stents or peripheral stents, surgical, dental or orthopedic implants, implantable orthopedic fixation aids, orthopedic bone prostheses or joint prostheses, artificial hearts or parts thereof, artificial heart valves, heart pacemaker casings or electrodes, subcutaneous and/or intramuscular implants, implantable drug-delivery devices, microchips, or implantable surgical needles, screws, nails, clips, or staples, or seed implants or the like. Typically, implants are made of solid materials, either polymers, ceramics or metals. To enable drug-delivery, implants can also be produced with porous surfaces or by using porous materials, wherein a drug may be included in the pore system for *in vivo* release.

Any suitable implant material may be used in the manufacture of the implants of the present invention. For example, the implant can be made from metal or metal alloys, e.g., selected from main group metals of the periodic system, transition metals such as copper, gold and silver, titanium, zirconium, hafnium, vanadium, niobium, tantalum, chromium, molybdenum, tungsten, manganese, rhenium, iron, cobalt, nickel, ruthenium, rhodium, palladium, osmium, iridium or platinum, or from rare earth metals. The material can also be made from organic materials, e.g., thermoset or thermoplastic polymers, synthetic rubbers, extrudable polymers, injection molding polymers, moldable polymers, spinnable, weaveable and knittable polymers, oligomers or pre-polymerizes forms or mixtures thereof. Suitable polymers include, e.g., hompopolymers, copolymers, prepolymeric forms and/or oligomers of poly(meth)acrylate, unsaturated polyester, saturated polyester, polyolefines like polyethylene, polypropylene, polybutylene, alkyd resins, epoxy-polymers or resins, phenoxy polymers or resins, phenol polymers or resins, polyamide, polyimide, polyetherimide, polyamideimide, polyesterimide, polyester-amideimide, polyurethane, polycarbonate, polystyrene, polyphenole, polyvinylester, polysilicone, polyacetale, cellulosic acetate, polyvinylchloride, polyvinylacetate, polyvinylalcohol, polysulfone, polyphenylsulfone, polyethersulfone, polyketone, polyetherketone, polybenzimidazole, polybenzoxazole, polybenzthiazole, polyfluoro-carbons, polyphenylenether, polyarylate, cyanatoester-polymere, and mixtures thereof.

Magnetic particle imaging (MPI) allows direct 3D imaging of magnetic particles, i.e., MPI tracer agents. Within the context of the present invention, the term "magnetic particles" also refers to "magnetizable particles". Spatial images are produced by measuring the magnetic fields generated by the MPI tracer agents introduced in an examination area. Magnetic particle imaging methods are disclosed, e.g., in DE 10151778 or US 2006/0210986. As described in said documents, it has been found that the change in the spatial distribution of the magnetic particles can be determined in the examination area, wherein the examination area can be present in living tissue or living organisms such as microorganisms, plants or mammals. For example, the change in the spatial distribution of the magnetic particles can be correlated with a local concentration, pressure, shear, viscosity, temperature and/or a local pH value. Furthermore, the particles are enriched to a different extent in different types of tissue. This effect enables so-called "molecular imaging" by visualizing the distribution of such particles and can be used, e.g., for monitoring development, remodeling or degradation of tissue.

MPI tracer agents which are suitable for the embodiments of the present invention are described, e.g., in WO 2004/091397, US 2007/0036729, DE 10151778 or DE 10238853. The MPI tracer agents may have a suitable size and shape. In an exemplary embodiment, the MPI tracer agent is dimensioned such that only a single magnetic domain (the monodomain) can form therein and there are no white regions. According to a one variant of the invention, suitable particle sizes lie in the range from 5 nm to around 5000 nm, 10 to 800 nm, or 20 to 50 nm. Typically, the upper limit depends on the material used.

For the embodiments of the present invention, it is a necessary prerequisite that the particles are biocompatible. Suitable materials of the MPI tracer agent can comprise magnetite (Fe₃O₄), maghemite (γ-Fe₂O₃) and/or non-stoichiometric iron oxides or mixtures thereof. In addition, further metal oxides can be added to the iron oxides, said metal oxides preferably being selected from magnesium, zinc and cobalt. These further metal oxides may be added to the iron oxide in proportions of up to 20% in total. Furthermore, manganese, nickel, copper, barium, strontium, chromium, lanthanum, gadolinium, europium, dysprosium, holmium, ytterbium and samarium can be contained in quantities of less than 5%, preferably less than 1%. However, any other suitable magnetic or magnetizable material can be used.

The biocompatibility of the MPI tracer agent can be further improved or modified by encapsulating the magnetic material. In one embodiment, the at least one MPI tracer agent can be surrounded by a pharmaceutically acceptable shell. In an exemplary embodiment, the substance or substance mixture the pharmaceutically acceptable shell is made of is biodegradable, and, e.g., can be cleaved into small units that can be used and/or can be removed by the living organism. A plurality of such substances and methods for producing them are described in the prior art, e.g., in US 5492814 or US 2007/0036729. The pharmaceutically acceptable shell can comprise, e.g., synthetic polymers or copolymers, starch, dextran, cyclodextran, fatty acids, polysaccharides, lecithin, mono-, di- or triglycerides, proteins or polypeptides or a mixtures thereof.

In one embodiment, the at least one MPI tracer agent surrounded by a pharmaceutically acceptable shell further comprises at least one ligand which is immobilized on the surface of the shell. This can provide a considerable increase in affinity or specificity to the biocompatible material, in which the MPI tracer agent is incorporated or adhered to. By way of example, a polysaccharides, e.g. dextran, or polymer, e.g. polyvinyl alcohol, can be used as ligand.

In another embodiment of the present invention, the at least one MPI tracer agent can comprise a non-magnetic nucleus and a coating consisting of a magnetic material such as described above. In an exemplary embodiment, the non-magnetic nucleus can comprise physiologically acceptable natural materials, e.g., silica or latex.

The biocompatible material comprising at least one MPI tracer agent can be synthesized using any suitable method that is known in the art. Examples for applicable methods are nanofiber self-assembly, textile technologies such as wet or dry spinning methods, electro-spinning, knitting or weaving, as well as solvent casting, or emulsification/freeze-drying. Alternatively or additionally, suitable bulk materials may be structured in a suitable way by folding, embossing, punching, pressing, extruding, gathering, injection moulding and the like before or after being moulded or formed.

Textile technologies may include all the approaches that have been successfully employed for the preparation of non-woven meshes of different polymers. By way of example, it is refered to the process of electrospinning, which involves the creation of an electrical field at the surface of the liquid comprising at least one polymer. The resulting electrical force creates a jet of liquid which carries electrical charge. The liquid jets may be attracted to other electrically charged objects at a suitable electrical potential and as the jet of liquid elongates and travels, it will harden and dry. A number of examples for the manufacture of porous materials that are suitable, e.g. as artificial tissue constructs, using electrospinning can be found in US 2006/0204445.

In one embodiment, the at least one MPI tracer agent is incorporated in the biocompatible product. For example, the at least one MPI tracing agent may be embedded, entrapped or entangled within the biocompatible product or contained within cavities, enclosures, inclusions or pockets thereof. The at least one MPI tracer agent can be incorporated into the biocompatible product by mixing, blending or melding with the starting material employed in the corresponding method for manufacturing the biocompatible product. Alternatively, the biocompatible product can be loaded with the at least one MPI tracer agent subsequently, e.g., by dipping the biocompatible product into a bulk material containing the at least one MPI tracing agent.

In another embodiment, the at least one MPI tracer agent is adhered to the surface of the biocompatible product. For example, the at least one MPI tracer agent can be bound to a specific site of the biocompatible product. In an exemplary embodiment, the MPI tracer agent can be adsorbed to the surface of the biocompatible product. In another exemplary embodiment, covalent bonds can be formed between at least one ligand immobilized on a pharmaceutically acceptable shell of the at least one MPI tracing agent and functional groups on the surface of the biocompatible product.

In one embodiment, at least one MPI tracer agent is used for visual monitoring a biocompatible product by magnetic particle imaging. In an exemplary embodiment, the at least one MPI tracer agent is used for monitoring development, remodelling and degradation of an artificial tissue construct. For example, magnetic particle imaging (MPI) can be used for quantification of the local concentration of a tracer agent. Without wishing to be bound to any theory, the inventors believe that biodegradation and remodelling result in 'ablation', i.e. removal of the MPI tracer agent, which can be monitored by MPI signal reduction over time. Remodelling may also result in modification of the 3D image structure itself. The monitoring can be carried out *in vitro* or *in vivo.* For example, vascular and tissue organ constructs can be monitored.

In another exemplary embodiment, implants which have been functionalized or blended with at least one MPI tracer agent are visualized using magnetic particle imaging.

In another embodiment, the at least one MPI tracer agent is used for substance delivery tracking and monitoring. Suitable therapeutic agents or materials for tissue engineering are, e.g., cells, biomedicals or pharmaceuticals. In still another embodiment, the at least one MPI tracer agent is used for visualization of targeted delivery of cells utilizing microcontainers loaded with cells.

### EXAMPLES

### Example 1- Manufacture of a degradable scaffold containing a MPI tracer agent

45 wt.-% collagen I, 15 wt.-% elastin and 40 wt.-% poly(lactic-co-glycolic acid) (PLGA) are blended with iron oxide magnetic nanoparticles having a size of about 30 nm. The solutes are dissolved in 1,1,1,3,3,3-hexafluoro-2-propanol at a total concentration of 10w/v % (100 mg/ml). The obtained solution is subjected to electospinning. A highly porous scaffold is obtained. High molecular weight PLGA can be added to the solution to increase mechanical strength of the scaffold. Scaffolds have typical thickness of 1mm and lateral size of several centimeters.

### Example 2 - Manufacture of a bilayer microcontainer containing a MPI tracer agent

Ebecryl 1810, supplemented with 0.1 wt% initiator (Irgacure 651) and 1:10 initiator: inhibitor (4-methoxyphenol), is spincoated on a glass substrate (treated with 10 minutes UV-ozone) at 3000 RPM for 30 seconds. Subsequently the substrate is positioned beneath two masks, one squared and one striped, and irradiated for 1 second in the UV-setup UV-2 with filter. After rinsing with isopropylalcohol a pattern is obtained.

Subsequently, the glass plate with patterned Ebecryl layer is used as one of two substrates to make a cell as formed by two plan parallel substrates. The patterned Ebecryl layer is positioned on the inside of the cell. The spacing of the cell is set to be 50 µm by using 50 µm thick tape as spacers and the cell is filled via capillary forces with a reactive mixture consisting of 50 wt.-% n-isopropylacrylamide (NIPAA) (1 mol% diethyleneglycoldiacrylate (DEGDA)) blended with iron oxide magnetic nanoparticles having a size of about 30 nm in 50/50 wt.-% water/methanol. The cell is placed beneath a shadow mask (squared features) and irradiated with UV -light for 4 minutes. After irradiating the cell is opened by removing the top substrate and rinsed with water. Then the bottom substrate, containing the polymerized composite elements forming the flakes, is placed in a water bath at room temperature. At roomtemperature the PNIPAA hydrogel shows strong swelling in water and as a result the patterned flakes were released from the substrate and rolled up in the direction perpendicular to the Ebecryl 1810 stripes. Upon heating the water bath above 33°C the PNIPAA hydrogel collapses (PNIPAA has a LCST at 33°C) and the hydrogel layer shrinks. As a result, the bi-layers unrolled upon heating the water bath above 33°C.

## Claims

1. Use of a MPI tracer agent for visual monitoring a biocompatible product comprising at least one magnetic particle imaging (MPI) tracer agent using magnetic particle imaging, wherein the biocompatible product is selected from the group consisting of a microcontainer comprising a rigid or semi-rigid material, an artificial tissue construct and an implant.

2. The use of claim 1, wherein the artificial tissue construct is a scaffold.

3. The use of claim 1, wherein the microcontainer comprises a porous material.

4. The use of claim 1, wherein the implant is selected from the group consisting of vascular endoprostheses, intraluminal endoprostheses, stents surgical, dental or orthopedic implants, implantable orthopedic fixation aids, orthopedic bone prostheses or joint prostheses, artificial hearts or parts thereof, artificial heart valves, heart pacemaker casings or electrodes, subcutaneous and/or intramuscular implants, implantable drug- delivery devices, microchips, or implantable surgical needles, screws, nails, clips, or staples, or seed implants.

5. The use of any one of the preceding claims, wherein the at least one MPI tracer agent is incorporated into the biocompatible product.

6. The use of any one of the preceding claims, wherein the at least one MPI tracer agent is adhered to the surface of the biocompatible product.

7. The use of any one of the preceding claims, wherein the at least one MPI tracer agent is surrounded by a pharmaceutically acceptable shell.

8. The use of claim 7, wherein the at least one MPI tracer agent surrounded by a pharmaceutically acceptable shell further comprises at least one ligand which is immobilized on the surface of the shell.

9. The use of any one of the preceding claims, wherein the at least one MPI tracer agent has a particle size between 5 and 5000 nm.

10. The use of claim 9, wherein the at least one MPI tracer agent has a particle size between 20 and 50 nm.

## Patentansprüche

1. Verwendung einer MPI-Tracersubstanz zur visuellen Überwachung eines biokompatiblen Produktes mit mindestens einer Tracersubstanz für Magnetpartikeltomographie (Magnetic Particle Imaging, MPI) mithilfe der Magnetpartikeltomographie, wobei das biokompatible Produkt aus der Gruppe bestehend aus einem Mikrocontainer, der ein steifes oder halbsteifes Material umfasst, einem künstliches Gewebekonstrukt und einem Implantat ausgewählt wird.

2. Verwendung nach Anspruch 1, wobei das künstliche Gewebekonstrukt ein Scaffold (Zellträger) ist.

3. Verwendung nach Anspruch 1, wobei der Mikrocontainer ein poröses Material umfasst.

4. Verwendung nach Anspruch 1, wobei das Implantat aus der Gruppe bestehend aus vaskulären Endoprothesen, intraluminalen Endoprothesen, Stents, chirurgischen Implantaten, Zahn- oder orthopädischen Implantaten, implantierbaren orthopädischen Befestigungshilfen, orthopädischen Knochenprothesen oder Gelenkprothesen, künstlichen Herzen oder Teilen davon, künstlichen Herzklappen, Herzschrittmachergehäusen oder -elektroden, subkutanen und/oder intramuskulären Implantaten, implantierbaren Arzneimittelverabreichungsvorrichtungen, Mikrochips oder implantierbaren chirurgischen Nadeln, Schrauben, Nägeln, Klemmen oder Klammern oder Seed-Implantaten ausgewählt wird.

5. Verwendung nach einem der vorhergehenden Ansprüche, wobei die mindestens eine MPI-Tracersubstanz in das biokompatible Produkt integriert ist.

6. Verwendung nach einem der vorhergehenden Ansprüche, wobei die mindestens eine MPI-Tracersubstanz an der Oberfläche des biokompatiblen Produktes haftet.

7. Verwendung nach einem der vorhergehenden Ansprüche, wobei die mindestens eine MPI-Tracersubstanz von einer pharmazeutisch annehmbaren Hülle umgeben ist.

8. Verwendung nach Anspruch 7, wobei die mindestens eine von einer pharmazeutisch annehmbaren Hülle umgebene MPI-Tracersubstanz ferner mindestens einen Liganden umfasst, der auf der Oberfläche der Hülle immobilisiert ist.

9. Verwendung nach einem der vorhergehenden Ansprüche, wobei die mindestens eine MPI-Tracersubstanz eine Partikelgröße zwischen 5 und 5000 nm hat.

10. Verwendung nach Anspruch 9, wobei die mindestens eine MPI-Tracersubstanz eine Partikelgröße zwischen 20 und 50 nm hat.

## Revendications

1. Utilisation d'un agent traceur de MPI pour la surveillance visuelle d'un produit biocompatible comprenant au moins un agent traceur d'imagerie par particules magnétiques (« Magnetic Particle Imaging » ou MPI) en utilisant une imagerie par particules magnétique, dans laquelle le produit biocompatible est sélectionné parmi le groupe constitué d'un micro-contenant comprenant un matériau rigide ou semi-rigide, d'une construction tissulaire artificielle et d'un implant.

2. Utilisation selon la revendication 1, dans laquelle la construction tissulaire artificielle est un échafaudage.

3. Utilisation selon la revendication 1, dans laquelle le micro-contenant comprend un matériau poreux.

4. Utilisation selon la revendication 1, dans laquelle l'implant est sélectionné parmi le groupe constitué d'endoprothèses vasculaires, d'endoprothèses intra-cavitaires, de stents, d'implants chirurgicaux, dentaux ou orthopédiques, d'aides de fixation orthopédiques implantables, de prothèses osseuses orthopédiques ou prothèses articulaires, de coeurs artificiels ou parties de ceux-ci, de valvules cardiaques artificielles, de boîtiers ou électrodes de stimulateur cardiaque, d'implants sous-cutanés et/ou intramusculaires, de dispositifs implantables d'administration de médicament, de micro-puces, ou d'aiguilles, de vis, de clous, d'attaches, ou d'agrafes chirurgicaux implantables, ou d'implants d'inoculation.

5. Utilisation selon une quelconque des revendications précédentes, dans laquelle l'au moins un agent traceur de MPI est incorporé dans le produit biocompatible.

6. Utilisation selon une quelconque des revendications précédentes, dans laquelle l'au moins un agent traceur de MPI adhère à la surface du produit biocompatible.

7. Utilisation selon une quelconque des revendications précédentes, dans laquelle l'au moins un agent traceur de MPI est entouré par une enveloppe pharmaceutiquement acceptable.

8. Utilisation selon la revendication 7, dans laquelle l'au moins un agent traceur de MPI entouré par une enveloppe pharmaceutiquement acceptable comprend en outre au moins un ligand qui est immobilisé sur la surface de l'enveloppe.

9. Utilisation selon une quelconque des revendications précédentes, dans laquelle l'au moins un agent traceur de MPI présente une taille des particules entre 5 et 5000 nm.

10. Utilisation selon la revendication 9, dans laquelle l'au moins un agent traceur de MPI présente une taille des particules entre 20 et 50 nm.
